Europäisches Patentamt

(19) European Patent Office

Office européen des brevets

(11) Publication number: **0 051 941**
**A1**

## EUROPEAN PATENT APPLICATION

(21) Application number: 81304946.7

(22) Date of filing: **21.10.81**

(51) Int. Cl.³: **C 02 F 3/28**, A 01 C 3/02, **F 03 D 9/00**

(30) Priority: **07.11.80 GB 8035780**

(43) Date of publication of application: **19.05.82** Bulletin 82/20

(84) Designated Contracting States: **AT BE CH DE FR GB IT LI NL SE**

(71) Applicant: **Evans, David Wynford, Caerfai Farm, St. David's Haverfordwest, Dyfed Wales (GB)**

(72) Inventor: **Evans, David Wynford, Caerfai Farm, St. David's Haverfordwest, Dyfed Wales (GB)**

(74) Representative: **Silverman, Warren et al, HASELTINE LAKE & CO. Hazlitt House 28 Southampton Buildings Chancery Lane, London WC2A 1AT (GB)**

(54) Anaerobic digester.

(57) An anaerobic digester for use in the treatment of farmyard waste to yield a methane-containing gas and a liquid fertiliser comprises a covered elongate tank (1) to one end (13) of which raw slurry is fed and from the other end (15) of which treated slurry is removed. Positioned within the tank (1) towards the slurry entry end thereof is a vertically arranged heat exchanger (18) comprising a cylindrical body of annular cross-section and up through which slurry is drawn by rotatable means (35), usually paddles carried on a shaft (23) connected to drive means (24) for effecting rotation thereof. Adequate power supply in open country locations can generally be achieved by use of a windmill as drive means (24).

- 1 -

"ANAEROBIC DIGESTER"

This invention relates to an anaerobic digester for use in the treatment of farmyard waste to yield methane as a source of energy available on site.

In recent years, an increasing amount of interest in anaerobic digesters for the treatment of farmyard waste and in particular cow and pig slurries to produce methane as a source of energy for use on the farm has taken place. These digesters generally take the form of large steel structures positioned above ground where they present something of an eyesore and moreover require substantial capital expenditure and energy input in respect of ancillary equipment such as pumping systems for transferring the slurry thereinto. In addition, because their walls are positioned entirely above ground, a particularly high standard of building construction is required when producing them.

Further problems associated with anaerobic digesters hitherto employed have frequently resulted from formation of crusts or matting on top of the slurry which needs to be removed for efficient gas release to occur. This crust removal can generally only be achieved by shutting down the digester.

Such problems are particularly exacerbated in anaerobic digesters used for the treatment of farmyard waste because of the substantial quantities of fibrous matter present in farmyard waste, consisting particularly of straw etc. present therein as a result of the farmyard waste being produced by "mucking out" under cover animal habitats as well as open farmyards themselves.

Whilst many forms of slurry digester have hitherto been described, they have generally been intended for processing of municipal sewage, having a main purpose to rapidly decontaminate and liquify the organic sewage before dumping it into streams and land fills. Because of the scale of operation required, such digesters tend to be of considerable size and in principle not suited for farm use, notwithstanding their not being designed with a main object of producing a useful gas having a high methane content, as well as a high quality liquid fertiliser for manure. One such anaerobic digester, but of a smaller size which may be mentioned is that described in Swiss Patent Specification No. 229.194. This is however characteristically suitable only for use in sludge decomposition on a relatively small scale and is not suited for the treatment of farmyard waste with all the additional materials usually present therein.

One form of digester which has hitherto been described as being suitable for use in the treatment of farm manure to produce methane gas and a liquid fertiliser is described in United States Patent Specification No. 4,100,023. With this arrangement, there is provided a trench dug in the ground and provided with a vertical access hole dug into the ground adjacent the front edge of the trench and connected thereto through a tunnel, the trench being occupied by a pillow of elastomeric material which is intended to house slurry in the lower part thereof within the trench and to be inflated in the upper part thereof by gases produced on digestion of the slurry. The lower portion of the pillow is separated by dividers made of the same flexible material as the pillow and joined to the pillow in a complicated arrangement of component parts ensuring that semi-rigid partitions result. Through tubes formed through the partitions serve to intercommunicate the individual chambers. Supply of raw slurry is through piping passing down through the access hole and embedded in gravel passing through the tunnel, in which gravel are also embedded output line and a digest recycle line. A coil heat exchanger is provided in the middle chamber. This complicated construction of the digester is with the view to providing temperature conditions at various stages in the decomposition of the slurry suited to the bacteria operating at such stages. There is no special provision for avoiding crust formation and the system would appear to be intended for use only with manure as such which will not contain the large amounts of fibrous waste mentioned already herein and which will give rise to clogging of the piping systems employed for supply and removal of slurry and for transfer of slurry between the chambers within the system. Transfer of slurry within the system is intended to be purely as a displacement operation wherein the periodical feeding of a load of raw slurry to the first chamber results in an equal volume of slurry being automatically transferred from each chamber to the next and to the output line.

It is an object of this invention to provide an anaerobic digester capable of handling substantial quantities of farmyard waste including much fibrous matter to produce a liquid fertiliser and a useful gas comprising methane.

It is a further object of this invention to provide an anaerobic digester meeting the first object of the invention and having a particularly low requirement in operation for energy input from an external source and which minimises crust formation when the digester is in use.

According to the present invention, there is provided an anaerobic digester for use in the treatment of farmyard waste to yield a methane-containing gas and liquid fertiliser, which comprises an elongate tank, cover means for the tank, inlet means for feeding slurry to one end of the tank and outlet means for removal of slurry from the other end of the tank, heat exchanger means within the tank having associated conduit means for supply of the heated heat exchange fluid thereto and for removal of heat exchange fluid therefrom, and outlet means in an upper region of the tank for removal of methane produced in the tank, the heat exchange means being positioned nearer to the inlet means for slurry into the tank than the outlet means therefrom and comprising a cylindrical body of annular cross-section having its bottom end spaced above the base of the tank and providing a through passage through the cylindrical interior thereof for the slurry, the cylindrical interior of the heat exchanger containing therewith rotatable means positioned so that rotation thereof causes slurry to be drawn up the cylindrical interior of the heat exchanger to be heated by heat exchange fluid passed through the cylindrical body, the rotatable means being carried on a shaft connected to drive means for effecting rotation thereof.

By providing a heat exchanger for the slurry within the tank in a cylindrical form with a rotatable member as aforesaid therein, the slurry undergoes simultaneously heating to the required temperature of 32 to 35°C in a continuous upward flow such that the formation of a crust on the top of the slurry and in a wide zone therearound is prevented. Although the rotatable means will generally preferably comprise paddles carried by a shaft and will be described herein with respect to such a construction, this invention is not limited to use of such means within the heat exchanger.

In a preferred construction of an anaerobic digester embodying this invention, the tank is set partly or completely below ground level. This provides a number of advantages. Firstly it enables a conventional lagoon to be employed to support side walls of the tank. The tank is then at the level of the lagoon and adjacent the remainder thereof facilitating ease of transfer of spent slurry to the remainder of the lagoon for subsequent transfer to areas to be cultivated for spreading thereover as fertilizer. An advantage of particular value insofar as minimizing or obviating the need for pumping equipment to supply the slurry to the tank is concerned is the fact that the slurry can simply be swept from barn, byre or sty into an inlet chamber positioned adjacent the tank and out of which slurry is able to flow directly into the tank. Preferably an upwardly sloping concrete ramp is positioned adjacent this chamber along which slurry may be scraped and in which may be situated a stone trap for ensuring that only stone free slurry is fed into the digester.

Although the rotatable means within the heat exchanger may be driven by for example a hydraulic motor, it has been found that a windmill will generally be a sufficiently vigorous source of power for the shaft, bearing in mind the generally exposed location of farms. Windmills are relatively inexpensive to construct, have a free source of energy and generally require minimum maintenance.

A shaft driven by the windmill can comprise gearing arrangements carrying drive means to ancillary rotatable equipment which may be incorporated in or used in association with the digester as will be described hereinafter with respect to the drawings.

For a better understanding of the invention, and to show how the same may be carried into effect, reference will now be made, by way of example only, to the drawings wherein:-

FIGURE 1 is a vertical section through an anaerobic digester embodying this invention;

FIGURE 2 is a plan view of the digester of Figure 1;

FIGURE 3 is a plan view of a heat exchanger within the tank of the digester;

FIGURE 4 is a plan view of an auxiliary stirrer within the digester; and

FIGURE 5 is the vertical section through the portion of the digester comprising the heat exchanger shown in Figure 1, but to a larger scale;

FIGURE 6 is a section taken at right angles to that shown in Figure 5;

FIGURE 7 is the vertical section through a further portion of the digester as shown in Figure 1, reproduced to a larger scale;

FIGURE 8 is a vertical section through a form of gas storage tank arrangement for use with an anaerobic digester embodying this invention; and

FIGURE 9 is a plan view of the gas storage tank of Figure 8.

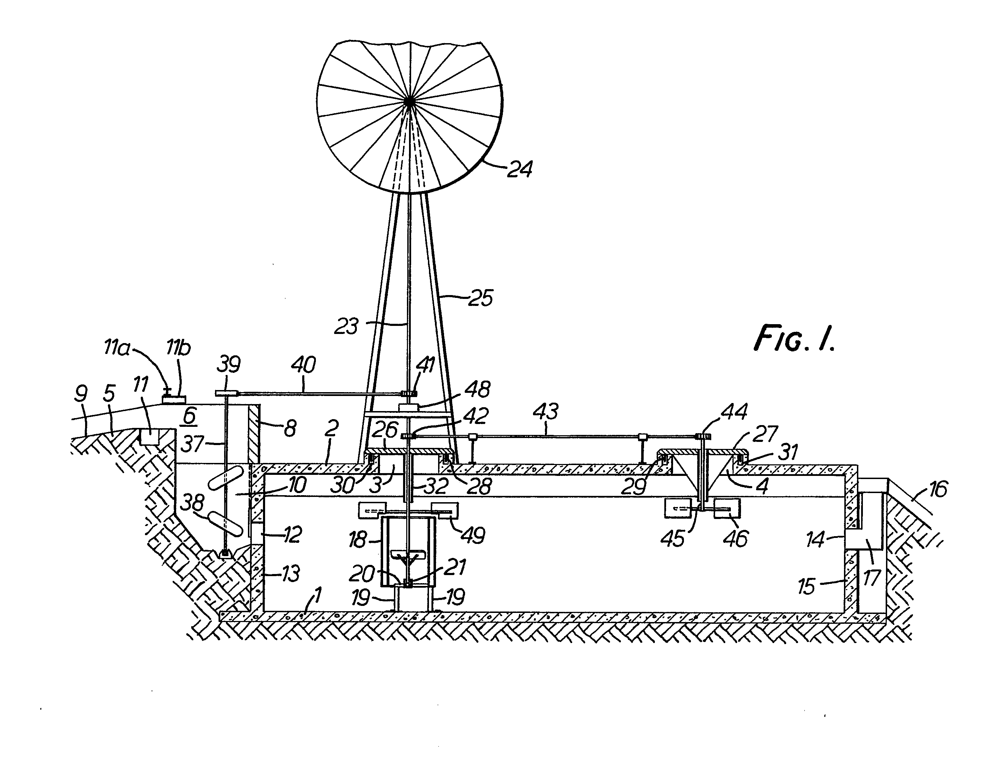

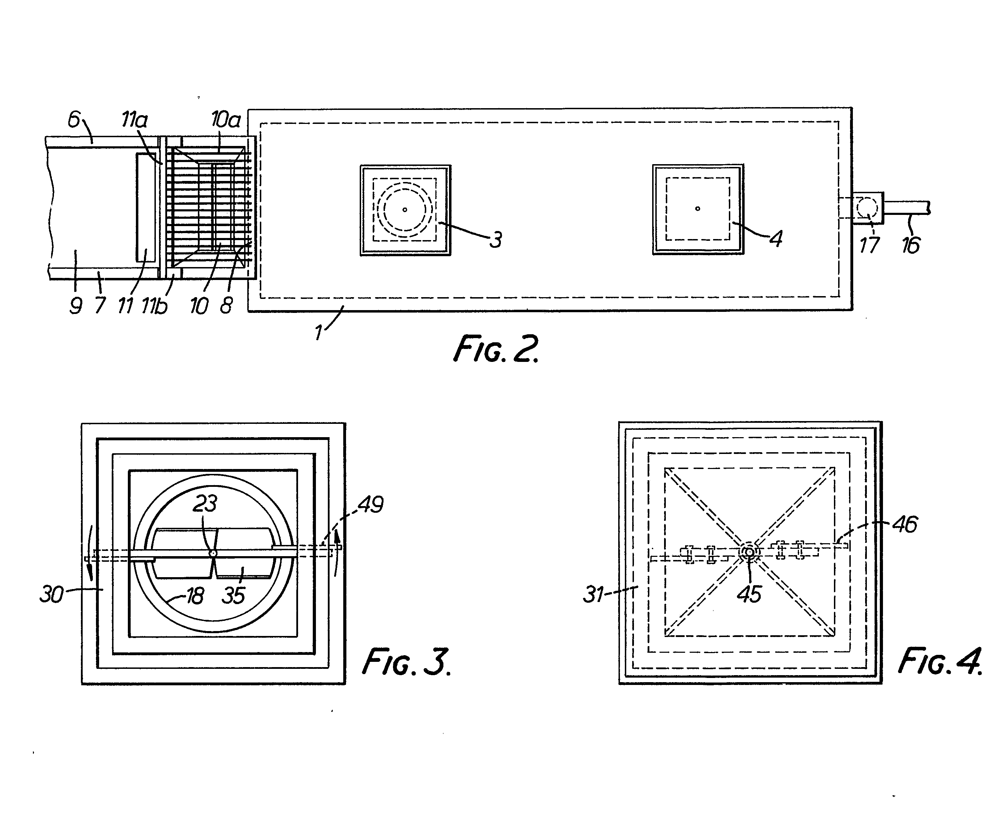

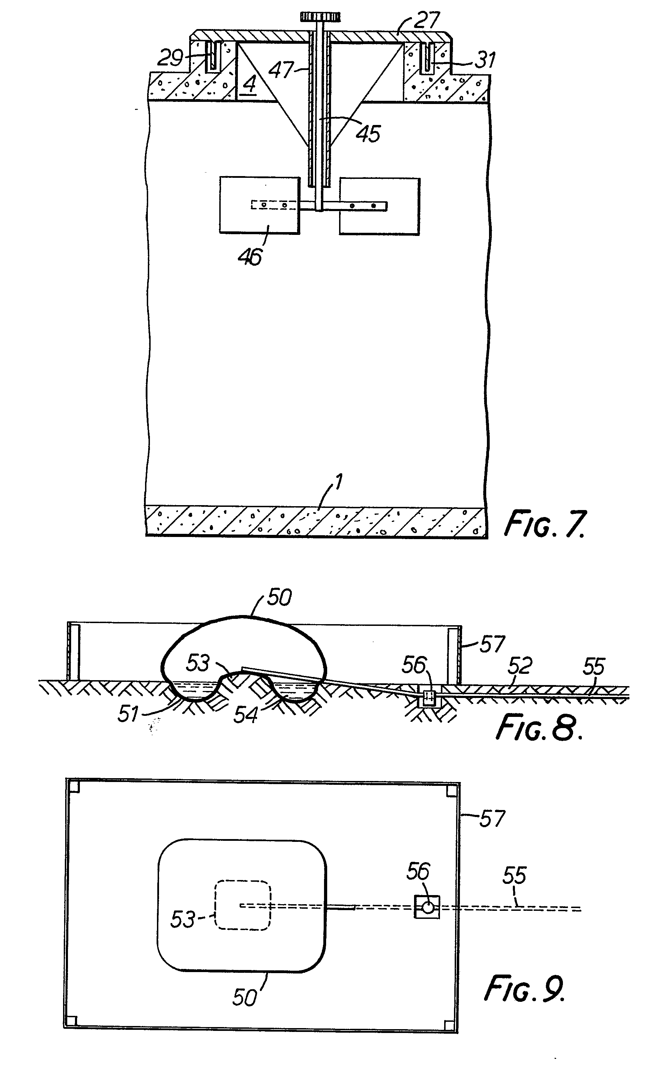

Referring to Figures 1 to 7 of the drawings, there is shown an anaerobic digester positioned partially below ground level and comprising a tank 1 constructed of reinforced concrete and typically having the dimensions 9.9 metres x 3 metres x 2.4 metres in depth. The tank comprises a roof 2 in which are formed square openings 3 and 4 equidistantly spaced from the ends of the digester. These provide access for desilting and for the installation of heat exchange means to be described hereinafter. Thermal insulation (not shown) is provided by means of the product "Styrofoam SM" (Registered Trademark) employed in a thickness of say 2.5 cm at the base and 5 cm at the roof and externally on the outside walls. Further insulation results from the digester being partially embedded in the ground. Additional insulation or protection from cold winds results from the digester being surrounded by an earth embankment 5 on which is provided a pair of parallel walls 6 and 7 (Figure 2) leading to an end wall 8 and delimiting an upwardly sloping concrete ramp 9 up which slurry may be pushed using a tractor and yard scraper to supply it to a catchment pit 10 covered with a grid 10a constructed from steel rods extending in the direction towards the top of wall 8. A gap of about 10 cm exists between each rod. The grid prevents the entry into the trap of further non-digestible objects. A stone trap 11 in the floor of the path 9 ensures that heavy solid material does not reach the tank 1. A further safety means is a tractor stop 11a positioned between stone trap 11 and catchment pit 10. This comprises a horizontal R.S.J. beam positioned about 60 cm above the ramp 9 and supported by two piles 11b set in the tops of the walls 6 and 7. A wide opening 12 midway up an end wall 13 of the tank communicates the catchment pit with the interior of the tank while an opening 14 in the opposite end wall 15 enables spent slurry to leave the tank and pass through a pipe 16 to a lagoon (not shown). Entry to the pipe 16 is through an L-shaped shaft 17 by which the liquid level in the digester may be ascertained.

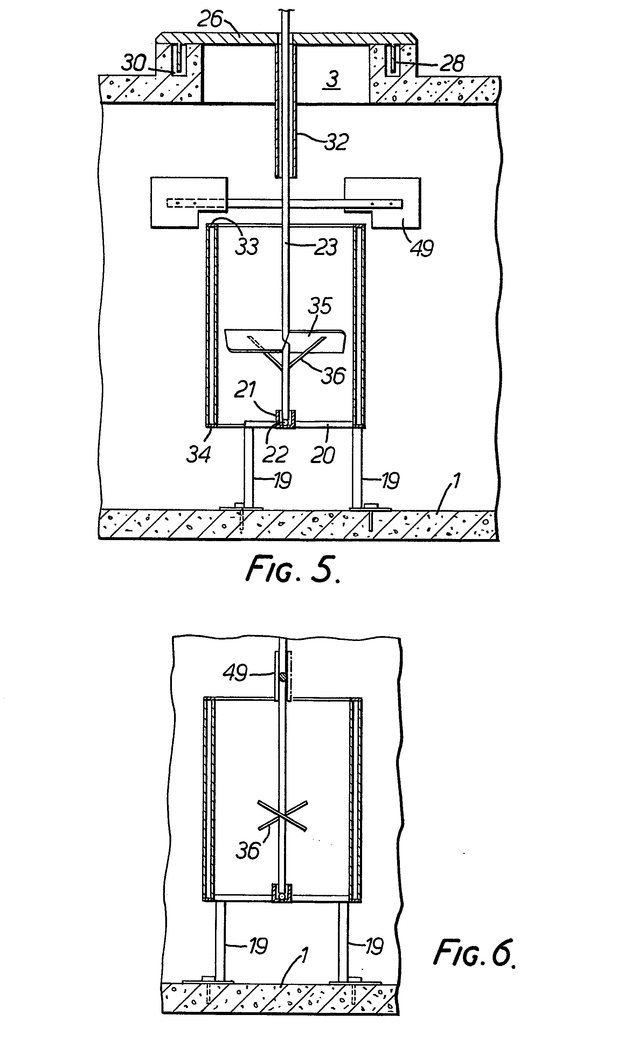

A heat exchanger 18 is positioned in the tank 1 beneath the opening 3. This heat exchanger has the form of a double walled cylinder formed by two concentric steel tubes welded to annular plates 33 and 34 at top and bottom (Figure 5) and whose cylinder interior is open at each end. The cylinder is supported above the base of the tank 1 by means of legs 19 and has positioned in the lower end thereof radial struts 20 carrying at the centre of the housing a locating tube 21 for a steel sphere 22 (Figures 5 and 6) welded to the end of a vertical shaft 23 which is subject to rotational motion imparted thereto by means of a windmill 24 mounted on a tower 25 above the square opening 3 and which passes through a manhole cover 26 over the opening 3. The steel sphere forms a bottom bearing for the shaft 23 and is lubricated by the surrounding slurry. Gas is prevented from escaping from the digester at the opening 3 (and also the opening 4 which is provided with a manhole cover 27) by side skirts 28 and 29 on covers 26 and 27 respectively disposed in water troughs 30 and 31 around openings 3 and 4 respectively. A steel tube 32 surrounding the shaft 23 is welded to the manhole cover 26.

Galvanised steel inlet and outlet ducts (not shown) to the water jacket constituted by heat exchanger 18 equipped with a circulation pump (not shown) respectively bring to the heat exchanger and remove therefrom heated water so as to ensure that slurry which enters the tank 1 achieves the optimum temperature of 32 to 35$^{o}$C for anaerobic digestion to take place. The shape of the heat exchanger enables there to be provided a chamber up which slurry can be drawn by rotation of paddles 35 carried by the vertical shaft 23 on rods 36. This upward drawing of the slurry through the heat exchanger ensures that the exchange of heat takes place in a particularly efficient manner. Moreover, although not shown, part of the heat content of the heat exchange fluid, water, supplied to the heat exchanger 18, may be from slurry leaving the tank 1 by means of heat exchange means (not shown) through which pipe 16 passes.

Although energy sources other than a windmill may be employed to rotate the shaft 23, for example a hydraulic motor or, where a source of water power is available, even a water mill, with a windmill positioned over the heat exchanger in the manner indicated, a particularly simple means is provided for converting the energy source, wind power, to rotational energy driving the paddles 35. Moreover, the provision of a windmill in the illustrated manner enables provision to be made for the driving of ancillary equipment by surplus power produced by the windmill, in particularly simple manner. Thus, as shown in Figure 1, the catchment pit 10 houses a vertical shaft 37 on which are mounted a pair of obliquely disposed paddles 38 of the wobble plate type. One of the paddles is shown above the slurry level in the tank 1. It will in fact be below the slurry level in the pit 10 which will be higher than the slurry level in the tank 1 owing to the gas pressure in the latter. The shaft 37 is mounted in a gear box 39 driven by a horizontal shaft 40 which is rotatable on engagement with a gear wheel 41 carried by the shaft 23. In addition a gear wheel 42 carried at a lower position on the shaft 23 is engageable with a horizontal shaft 43 for driving a gear wheel 44 positioned above the square opening 4. The gear wheel 44 is carried by a shaft 45 at the foot of which are carried stirrer blades 46. The shaft 45 passes through a steel tube 47 welded to manhole cover 27. The various paddles will generally be of hinged construction to allow paddles of adequate length to be admitted through the manifolds. When the vanes of the windmill are rotated backwards (feathered) the hinges will fold so that the blades fold and can be removed.

It has been found that a windmill can provide drive to the shafts 37 and 45 as well as to the main shaft 23 under conditions generally encountered. Moreover, although not shown, a water circulation pump for heat exchange water can be driven from the windmill. A windmill has been constructed which has a rotor speed of 120 rpm in a wind of approximately 32 kph. An enclosed gear box (not shown) at the windmill head may therefore be provided which provides a speed increase on the drive shaft of 1.33 to 1. Near the base of the windmill tower 25 is a reducing gear box 48 of 5:1 ratio. It has been found that when the windmill is subject to such a wind, the return temperature of hot water supplied to the heat exchanger 18 for providing a slurry temperature of 32 to 35$^{\circ}$C thereadjacent drops to 35$^{\circ}$C with a wind of 32 kph instead of 45$^{\circ}$C when there is no wind, thus indicating the improved slurry displacement which must be taking place in the heat exchanger and accordingly reduction in tendency to crust formation on the slurry above the heat exchanger. Moreover, as a consequence of

0051941

constructing the windmill arrangement in this manner, the resulting rotational speed of the paddles 35 of the agitator arrangement within the heat exchanger 18 varies from 10 rpm in a slight breeze of 13 to 16 kph to 35 rpm in a 48 kph wind. Power from the wind is estimated to be 745.7 W in a 24 kph wind to above 2250 W at 48 kph (above 48 kph, the mill feathers). In a strong breeze, there is a surplus power available from the mill and it is this power which will be available to drive shafts 37 and 45 carrying paddles 38 and stirrer blades 46 respectively. Depending upon the power available and particular requirements, it may only be desired to drive one of these stirring mechanisms at any particular time. In particular, there will only be need to drive the shaft 37 when the feed to the catchment pit 10 contains a lot of material which escapes the stone trap 11 and which it is desired to keep dispersed in the slurry in as finely a divided state as possible. This will be the case in particular when the slurry contains considerable amounts of straw, although cow slurries containing straw 10 to 12.5 cm, long have been found not to require use of paddles 38 or for that matter dilution or mixing before supply to tank 1. The stirrer blades 46 will be used to keep the outlet end of the digester tank free from crust build up. Crust build up in the region of the heat exchanger 18 will generally be prevented by the effect of the slurry rising upwards out of the heat exchanger. However, at those times when the heat exchanger and paddles 25 have not been operative, for example when servicing of the windmill has taken place, any crust which has formed in the region above the heat exchanger 18 may be broken up immediately recommencement of use of the windmill takes place by means of stirrer blades 49 which may be carried by the shaft 23. When a windmill as aforesaid is used under a 48 kph wind causing rotation of square paddle blades 49, the action of the blades is in fact sufficient to send ripples over most of the tank and obviates the need for paddles 45.

0051941

During use of the slurry digester, so-called biogas (about 65% methane) will be produced above the slurry. Gas lines for removal of this gas are not shown in the drawings in the interest of clarity. Gas outlets may be provided in manholes 26 and 27 over openings 3 and 4 and connected through 2.54 cm diameter flexible reinforced plastic pipe to the top of a gas holder (not shown). The limiting pressure here will be the height of the side skirts 28 and 29 (about 15 cm). Excess gas pressure will cause the manifold covers to be lifted and gas released to the atmosphere, rather than pressure buildup leading to an explosion. Another flexible pipe from the top of the gas holder can be employed to supply gas to a heating boilder for heating water to be supplied to the heat exchanger 18 and for supplying gas to boilers for providing hot water and central heating in adjacent buildings. A typical gas holder which is commercially available is one manufactured by "Farm Gas", being of glass fibre-reinforced plastics construction, having a capacity of 5663 litres gas working at 7.6 cm wg.

Should such a gas holder have insufficient capacity for the digester system, then the gas holder may be replaced by or supplemented by a particularly simple form of gas holder which is shown in Figures 8 and 9 of the accompanying drawings. The gas holder is formed of inflatable material, preferably butyl rubber. As illustrated, the gas holder comprises a casing 50 of substantially rectangular cross-section. The gas holder is located over a square channel 51 which may be provided in an area of ground 52. As shown, the ground is formed up into a mound 53 at the centre of the gas holder. The channel 52 is filled with water 54 up to ground level and it is the weight of this water which serves to hold the gas holder in position even when fully inflated. A gas duct 55 in which is situated a condensation trap 56 serves to supply gas to the holder. The duct 55 can be provided with a suitable valve (not shown) to enable gas also to be withdrawn therethrough to be supplied to the intended point of use. A gas holder of this type can readily be used for storing methane for driving internal combustion engines such as may be used on farms, where pressurized gas is not required. To comply with safety requirements, a perimeter fence 57 surrounds the gas holder. Typically, a butyl rubber casing gas holder of such type having a thickness of 0.076 cm and dimensioned 3.66 metres x 3.66 metres x 1.83 metres high will have a capacity of 22,650 to 25,500 litres when inflated.

In summary, a digester embodying this invention is capable of efficient operation with little maintenance. When a windmill is used as the prime power source the digester system can be set up and worked in locations where the average prevailing wind velocity is about 15 kph or more without the need for connection to mains electricity or any other source of motive power. The system will thus be of particular use in isolated country areas where there is a plentiful supply of animal (as well as human) and vegetable waste for digestion. The substantial energy saving achievable with a windmill may amount to 20 British units of electricity per day, this being the energy obtained with windmills when used as is normally the case for driving pumps.

- 14 -

A digester constructed as shown in the accompanying drawings has been found to work efficiently with slurry leaving the digester having a temperature of about 35°C. The value of the digester of the invention will be appreciated by the fact that when the source of slurry to the digester is a herd of 60 cows which is left outside for about 5 hours during the day, sufficient. slurry is available for gas production in the digester to be of the order of 4.5 cubic metres per hour, sufficient to supply simultaneously a 38,000 kJ boiler for digester heating, about 1.13 cubic metres per hour into a dual fueled engine, a 28,000 kJ boiler used in a dairy and a domestic cooker.

0051941

CLAIMS:

1.      An anaerobic digester for use in the treatment of farmyard waste to yield a methane-containing gas and a liquid fertiliser, which comprises an elongate tank, cover means for the tank, inlet means for feeding slurry to one end of the tank and outlet means for removal of slurry from the other end of the tank, heat exchanger means within the tank having associated conduit means for supply of heated heat exchange fluid thereto and for removal of heat exchanged fluid therefrom and outlet means in an upper region of the tank for removal of methane produced in the tank, characterised in that the heat exchanger means (18) is positioned nearer to the inlet means (12) for slurry into the tank than the outlet means (14) therefrom and comprises a cylindrical body of annular cross-section having its bottom end (20) spaced above the base of the tank (1) and providing a through passage through the cylindrical interior thereof for the slurry, the cylindrical interior of the heat exchanger containing therewithin rotatable means (35) positioned so that rotation thereof causes slurry to be drawn up the cylindrical interior of the heat exchanger to be heated by heat exchange fluid passed through the cylindrical body, the rotatable means (35) being carried on a shaft (23) connected to drive means (24) for effecting rotation thereof.

2.      A digester as claimed in claim 1, characterised by the presence of windmill driving means (24) for the shaft (23).

3.      A digester according to claim 2, characterised in that the rotatable means comprises paddles (35) carried by the shaft (23).

4.      A digester according to any one of the preceding claims, characterised further by paddle means (49) carried by the shaft (23) at a position above the heat exchanger means (18).

5.      A digester according to any one of the preceding claims, characterised by the presence of a gear wheel (42) at a position on the shaft (23) above the tank (1), a horizontal shaft (43) engaging with the gear wheel (42) for rotation thereby, a further gear wheel (44) acted on by the horizontal shaft (43), and a further shaft (45) entering the tank through an opening (4) positioned between the heat exchanger means (18) and the outlet means (14) from the tank (1), the further shaft (45) carrying the gear wheel (44) to be rotated thereby and additionally carrying at a position within the tank, paddle means (46).

6.      A digester according to claims 4 and 5, characterised in that the said paddle means comprise(s) paddles of square form.

7.      A digester according to any one of the preceding claims, characterised by the provision of a settling pit (10) positioned on the upstream side of the inlet means (12) to the tank (1), which inlet means (10) is an opening in an end wall (13) of the tank (1) communicating the settling pit with the tank for throughflow of farmyard waste supplied to the settling pit.

8.      A digester according to claim 7, characterised by a gear wheel (41) carried by the shaft (23) at a position thereon above the tank (1), the gear wheel (41) driving a horizontal shaft (40) which in turn drives a gear wheel (39) carried by a vertical shaft (37) entering the settling pit (10) and carrying paddle means (38) for effecting rotation thereof.

9.      A digester according to claim 7 or 8, characterised by the presence thereadjacent of an upwardly sloping concrete ramp (9) separated by a stone trap (11) from the mouth of the settling pit (10).

10.     A digester according to any one of the preceding claims, characterised in that the tank (1) is set partly or completely below ground level at the level of a lagoon positioned thereadjacent.

*Fig. 1.*

FIG. 2.

FIG. 3.

FIG.4.

0051941

FIG. 5.

FIG. 6.

4/4

**FIG. 7.**

**FIG. 8.**

**FIG. 9.**

European Patent Office

**EUROPEAN SEARCH REPORT**

0051941

Application number

EP 81 30 4946

| Category | Citation of document with indication, where appropriate, of relevant passages | Relevant to claim |
|---|---|---|
| D | US - A - 4 100 023 (B.A. McDONALD)<br><br>* column 2, line 54 - column 5, line 14 *<br><br>-- | 1,10 |
| D | CH - A - 229 194 (L. VON ROLL)<br><br>* page 2, lines 45-82 *<br><br>-- | 1 |
| P | US - A - 4 238 337 (M.F. PETERS et al.)<br><br>* column 2, lines 29-53 *<br><br>-- | 2,3,6 |
| A | US - A - 4 121 539 (J.T. MOORE) | |
| A | FR - A - 1 182 989 (HUMUGAZ)<br><br>----- | |

**DOCUMENTS CONSIDERED TO BE RELEVANT**

**CLASSIFICATION OF THE APPLICATION (Int. Cl. 3)**

C 02 F 3/28
A 01 C 3/02
F 03 D 9/00

**TECHNICAL FIELDS SEARCHED (Int.Cl. 3)**

C 02 F 3/28
A 01 C 3/00
A 01 C 3/02
C 12 M 1/00
F 03 D 9/00

**CATEGORY OF CITED DOCUMENTS**

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

The present search report has been drawn up for all claims

| Place of search | Date of completion of the search | Examiner |
|---|---|---|
| The Hague | 25-01-1982 | TEPLY |

EPO Form 1503.1   06.78